# EUROPÄISCHE PATENTSCHRIFT

(11) **EP 3 093 658 B1**
(45) Veröffentlichungstag und Bekanntmachung des Hinweises auf die Patenterteilung: **07.06.2023**
(21) Anmeldenummer: 16001058.3
(22) Anmeldetag: 11.05.2016
(51) Int. Cl.: G01N 30/12, G01N 30/16, G01N 30/72, G01N 30/08, G01N 30/24

(54) **VORRICHTUNG UND VERFAHREN DER GASANALYSE**
GAS ANALYSIS DEVICE AND METHOD
DISPOSITIF ET PROCÉDÉ D'ANALYSE DE GAZ

(30) Priorität: 11.05.2015 DE 102015005860
(43) Veröffentlichungstag der Anmeldung: 16.11.2016
(73) Patentinhaber: Fagus-GreCon Greten GmbH & Co. KG, 31061 Alfeld (DE)
(72) Erfinder: Schumann, Achim, 37154 Northeim (DE); Steckel, Vera, 30451 Hannover (DE); Hasener, Jörg, 31016 Alfeld (DE)
(74) Vertreter: Rieke, Andreas

(56) Entgegenhaltungen:
- EP-A1- 2 511 703
- EP-A2- 1 203 953
- WO-A2-2007/032039
- US-B1- 6 223 584
- "DIN ISO 16000-6 Innenraumluftverunreinigungen - Teil 6: Bestimmung von VOC in der Innenraumluft und in Prüfkammern, Probenahme auf Tenax TA, thermische Desorption und Gaschromatographie mit MS/FID (ISO/DIS 16000-6:2010)", DEUTSCHE NORMEN. DIN NORM,, Bd. 16000-6, Juni 2010 (2010-06), Seiten 1-40, XP009153689,
- SCHUMANN A ET AL: "Detection of volatile organic compounds from wood-based panels by gas chromatography-field asymmetric ion mobility spectrometry (GC-FAIMS)", INTERNATIONAL JOURNAL FOR ION MOBILITY SPECTROMETRY, SPRINGER-VERLAG, BERLIN/HEIDELBERG, Bd. 15, Nr. 3, 31. Mai 2012 (2012-05-31), Seiten 157-168, XP035106844, ISSN: 1865-4584, DOI: 10.1007/S12127-012-0103-3
- C. LENTH ET AL: "Developing a new method of measuring formaldehyde emissions from wood based panels", INTERNATIONAL WOOD PRODUCTS JOURNAL, Bd. 5, Nr. 3, 31. Juli 2014 (2014-07-31), Seiten 168-172, XP055300912, ISSN: 2042-6445, DOI: 10.1179/2042645314Y.0000000076

## Beschreibung

Die Erfindung betrifft eine Vorrichtung und ein Verfahren der Gasanalyse.

Zur qualitativen und quantitativen Bestimmung von Substanzen, die aus Materialien ausgasen, werden verschiedene Verfahren und Vorrichtungen verwendet. Die Auswahl von Verfahren und Vorrichtung richtet sich insbesondere nach den Eigenschaften der jeweiligen Substanz(en), wie z. B. ihrer Flüchtigkeit, und den Eigenschaften des jeweiligen Materials, wie z. B. seine Homogenität oder die Menge, die von einer bestimmten Substanz freigesetzt wird.

Materialien, die als Bauprodukte oder als Produkte zur Verwendung in Innenräumen zur Anwendung kommen, können zahlreiche Substanzen abgeben. Eine übliche Klassifizierung dieser Substanzen erfolgt über deren Siedebereich, demnach wird unterschieden zwischen
1. Very Volatile Organic Compound, VVOC,
   0 bis 50 ... 100°C
2. Volatile Organic Compound, VOC,
   50 ... 100 bis 240 ... 260°C
3. Semi Volatile Organic Compound, SVOC,
   240 ... 260 bis 380 ... 400°C

Zur Messung der Abgabe von Substanzen aus Materialien werden in der Regel Systeme verwendet, die sich zusammensetzen aus einer Einheit, die die kontrollierte Freisetzung der Substanzen ermöglicht, des weiteren einer Einheit, die das Sammeln der Zielsubstanzen erlaubt und ferner einer Einheit, mit der die Zielsubtanzen qualitativ und quantitativ bestimmt werden können. Im folgenden sollen verschiedene Systeme vorgestellt werden, die dem Stand der Technik entsprechen:
Zur Bestimmung der Formaldehydabgabe aus Holzwerkstoffen und anderen Holzprodukten werden Prüfkammermethoden angewendet, z. B. nach EN 717-1/ISO 1246-1. Hierbei werden Prüfkörper in eine Kammer eingebracht, die mit gereinigter und konditionierter Luft (Temperatur und relative Feuchte konstant) gespült wird. Luftdurchsatz, Kammervolumen, und emittierende Prüfkörperfläche sowie weitere Parameter sind festgelegt. Zur Beprobung wird eine definierte Luftmenge aus der Kammer durch ein Waschflaschenpaar geleitet, das mit destilliertem Wasser gefüllt ist. Hierbei wird der vom Prüfkörper abgegebene Formaldehyd aufgefangen. Die Lösung in den Waschflaschen wird zur Bildung eines Farbkomplexes (Hantzsche Reaktion) mit Chemikalien versetzt, und der Formaldehydgehalt spektrophotometrisch bei einer Wellenlänge von 412 mm bestimmt. Mit dieser sog. Acetyl-Aceton-Methode wird sehr selektiv ausschließlich Formaldehyd bestimmt, die Querempfindlichkeit für andere Stoffe, einschließlich Aldehyde, ist gering.

Mit Prüfkammermethoden wird die Formaldehydabgabe unter typischen/idealisierten Innenraumbedingungen nachgestellt (daher unterscheiden sich die Sollwerte für Temperatur, relative Luftfeuchte und ggf. Luftwechselrate je nach Region, für die die Norm gültig ist). Prüfkammermethoden sind Referenzmethoden, deren Prüfdauer mehrere Tage beträgt, z.B. weil die Prüfkörper vorkonditioniert werden oder weil bis zur Ausgleichskonzentration getestet wird. Davon abgeleitet wurden Methoden entwickelt, die innerhalb weniger Stunden und mit relativ geringem Aufwand Ergebnisse liefern und damit zur werkseigenen Produktionskontrolle der Hersteller geeignet sind. Die Ergebnisse der abgeleiteten Methoden korrelieren mit denen der Prüfkammermethoden. Dies ist zentral, da die Prüfkammermethode die Referenzmethode darstellt, nach der z. B. an Instituten gearbeitet wird, die die Fremdüberwachung von Herstellern durchführen und damit bescheinigen, dass die Grenzwerte der Formaldehydabgabe eingehalten werden. Zu den abgeleiteten Methoden gehört die Gasanalyse-Methode (DIN EN ISO 12460-3).

Bei der Gasanalyse-Methode werden Prüfkörper (400 mm x 50 mm x Plattenstärke des Holzwerkstoffs) gereinigter und getrockneter Luft mit einer Temperatur von 60 °C ausgesetzt. Durch die erhöhte Temperatur wird die Formaldehydabgabe beschleunigt und damit die Testdauer verringert. Der Luftstrom mit dem abgegebenen Formaldehyd wird aus dem Analysegerät jeweils für eine Stunde durch ein Wasserflaschenpaar (insg. vier Waschflaschenpaare) mit destilliertem Wasser geleitet. Die Waschflaschenpaare werden von außen mit dem Gasanalysegerät bzw. den dort befindlichen Ausgängen verbunden. Hierbei wird der vom Probenkörper abgegebene Formaldehyd aufgefangen, die Bestimmung erfolgt nach der Acetyl-Aceton-Methode wie bereits für die Prüfkammermethode beschrieben.

Zur Bestimmung der Abgabe einer ganzen Reihe von VOCs und SVOCs (im Bereich C₆ bis C₂₂) aus Materialien werden ebenfalls Prüfkammertests durchgeführt, jedoch unter geringfügig anderen Parametern als zur Bestimmung der Formaldehydabgabe.

Als Beispiel sei die ISO 16000-9 genannt. Zur Beprobung wird Luft aus der Prüfkammer und über ein Anreicherungsmedium gepumpt.

Das Anreicherungsmedium kann z. B. ein mit Adsorptionsmittel gefülltes Röhrchen sein(z. B. Tenax, Carbotrap), das axial von einem Gas durchströmt wird, bspw. in der US 6,223,584 B1 erläutert. Dieses in einer Abscheidevorrichtung angeordnete, beheizte Röhrchen wird nach Abschluss der Anreicherung für eine Auswertung von einer Transportvoricchtung umgesetzt.

Die Identifikation der Substanzen und ihre mengenmäßige Bestimmung erfolgt dann in der Regel mittels Gaschromatographie-Massenspektroskopie (GC-MS). Dazu werden die adsorbierten Substanzen per Thermodesorption (TD) in das GC-MS-System überführt. In der GC-Säule erfolgt die Auftrennung des komplexen Substanzgemischs, so dass die Substanzen einzeln in den Detektor (MS) gelangen.

Alternativ zur MS können Flammionisationsdetektoren (FID) eingesetzt werden, die jedoch eine deutlich höhere Bestimmungsgrenze aufweisen. ISO 16000-6 beschreibt beispielsweise die Probenahme auf Tenax und die anschließende Analytik mit TD-GC-MS. Mit dieser Methode ist es möglich, eine große Bandbreite von VOCs sowie einigen VVOCs und SVOCs zu erfassen. Sollen ausschließlich Aldehyde (einschließlich Formaldehyd) und Ketone bestimmt werden, können als Adsorptionsmittel Silikagel-Kartuschen verwendet werden, die mit einem Derivatisierungsmittel (DNPH) imprägniert wurden. Die Substanzen werden bei der Anreicherung derivatisiert. Die Derivatisierung zu Hydrazonenstabilisiert die Substanzen und ermöglicht die Bestimmung mit UV/VIS-Detektoren. Die Hydrazonewerden mit Acetonitril von den Kartuschen eluiert, und als Flüssigproben per High-Performance Liquid Chromatographie (HPLC) mit UV/VIS-Detektor bestimmt. Diese Methode wird z. B. in ISO 16000 Teil 3 beschrieben.

Um VVOCs im Bereich < C₆ zu bestimmen, wie z. B. Ameisen- oder Essigsäure, kann die Prüfkammerluft wie bei der Formaldehydbestimmung durch Waschflaschen geleitet und die Lösung mittels Ionenchromatographie analysiert werden. Ferner kommt auch für VVOCs die TD-GC-MS-Analytik zur Anwendung, jedoch mit anderen Adsorbentien als für VOCs bzw. SVOCs.

Wie bei der Formaldehydbestimmung mittels Prüfkammermethode erfordert auch die Bestimmung gasförmiger Substanzgemische, die von Materialien abgegeben werden, einen mehrtägigen Test in der Prüfkammer (z. B.28 Tage nach ISO 16000-9).Daher wurden auch für diese Anwendung vereinfachte bzw. beschleunigte Verfahren entwickelt. Im folgenden werden einige relevante Methoden vorgestellt:
Die Emissionsprüfzelle nach ISO 16000-10 wird zur Messung plattenförmiger Materialien verwendet, auf die sie mit ihrer Öffnung aufgesetzt wird. Dies ermöglicht auch eine Prüfung bereits verbauter Holzwerkstoffplatten. Die Anreichung der Prüfluft und die Bestimmung der Substanzen erfolgt nach ISO 16000-6 (Tenax mit TD-GC-MS). Eine Korrelation zur Prüfkammermethode ist gegeben.

Die Mikroprüfkammer (z. B. ISO 12219-3 oder ISO 16000-25) wird u. a. für Materialien eingesetzt, die zur Innenausstattung von Automobilen verwendet werden, aber auch für Lebensmittel. Es handelt sich um sehr kleine, temperierbare Prüfkammern mit einem Volumen von 40 bis 120 ml, in die die Prüfkörper eingebracht werden und die mit einem Trägergas gespült werden. Eine ähnliche Methode stellt die thermische Extraktion dar (VDA 278), hier werden ca. 8 cm lange Prüfköper in einen Glaszylinder mit einem Innendurchmesser von 13 mm eingebracht. Der Aufbau ist beheizbar und wird mit einem Trägergas gespült. Die Beprobung des mit abgegebenen Subtanzen beladenen Trägergases und die Analytik erfolgt für beide Methoden wie bei der herkömmlichen Prüfkammer (z. B. Probennahme auf Tenax, Analytik mittels TD-GC-MS). Ferner ist die Headspace-Analytik zu nennen, z. B. VDA 277 (nichtmetallische Werkstoffe der Kfz-Innenausstattung) mit einer Probeneinwaage von 5 ml bzw. 0,5 g. Hier wird aus dem Headspace über der Probe ein definiertes Probegasvolumen entnommen und direkt in ein GC-FID oder GC-MS eingebracht.

Zur Bestimmung von Gemischen flüchtiger organischer Verbindungen stehen damit ergänzend zur Prüfkammermethode mehrere vereinfachte bzw. beschleunigte Verfahren zur Verfügung. Jedoch bestehen zwei zentrale Nachteile: Zum einen ist die gleiche aufwendige und komplexe Analytik wie für die Prüfkammermethode erforderlich, zum andern sind die Methoden Mikroprüfkammer, thermische Extraktion und Headspace für Holzwerkstoffe bzw. Holzprodukte zwar für ein schnelles Screening, welche Substanzen in der Hauptsache abgegeben werden, einsetzbar, jedoch können mit den Ergebnissen keine Korrelationen zu Prüfkammerergebnissen aufgestellt werden. Ein wichtiger Grund hierfür ist die (extrem) geringe Probengröße, die für relativ inhomogenes Material wie Holzwerkstoffe/Holzprodukte die Verwendung repräsentativer Prüfkörper ausschließt. Ein weiterer Grund ist der hohe Beladungsgrad und die hohe Spül- oder Luftwechselrate, die deutlich von den Parametern in einer Prüfkammer abweichen.

Beide Nachteile (aufwendige Analytik und fehlende Korrelation zur Prüfkammermethode als Referenzmethode) verhindern eine Anwendung der genannten Verfahren in Werkslaboren von Herstellern von Holzwerkstoffen oder anderen Holzprodukten. Damit gibt es keine Möglichkeit zur werkeigenen Produktionskontrolle der Abgabe von VVOC, VOC und SVOC aus Produkten. Dies ist jedoch in der DIN CEN/TS 16516 (2013-12) gefordert. Alternativ können Prüfkörper an Prüfinstitute zur Durchführung der Tests verschickt werden. Dies hat jedoch den Nachteil, dass das Ergebnis erst nach mehreren Tagen vorliegt. Zudem hängen die Ergebnisse von Emissionstests in aller Regel stark vom Prüfzeitpunkt ab, d.h. um Ergebnisse vergleichen zu können, muss nicht nur der Probenahmezeitpunkt, sondern auch der Prüfzeitpunkt konstant gehalten werden. Eine Probenkonservierung durch luftdichte Verpackung ist nur eingeschränkt möglich.

Einen solchermaßen bekannten Stand der Technik erläutert bspw. die EP 2 511 703 A1. Aus dieser Druckschrift ist ein Verfahren für das Messen von emittierten flüchtigen Stoffen aus Holzwerkstoffen, insbesondere Formaldehyd, bekannt, bei dem von einem Holzwerkstoff einige Partikel als Messproben entnommen werden. Anschließend werden diese ionenmobilitätsspectrometrisch mittels eines FAIMS untersucht. Hierzu werden die von den Proben bei Raumtemperatur oder nach einem Erhitzen frei gesetzten Gase durch eine Adsorptionseinheit wie ein Adsorptionsröhrchen geleitet. Nach einer dortigen Anreicherung der flüchtigen Stoffe wird das Röhrchen kurzzeitig mit einer Ventilkombination beidseits geschlossen und möglichst schnell erhitzt. Nach einem Öffnen des Röhrchens und einer Umkehrung des Gasflusses erfolgt dann die Beaufschlagung einer gaschromatischen Säule, in der die Trennung der Stoffe erfolgt. Hieran schließt eine Detektierung der Stoffe mittels einer FAIMS-Anlage an.

Aufgabe der Erfindung ist es, eine Vorrichtung und ein Verfahren zur Verfügung zu stellen, die die Durchführung von Tests zur Abgabe von insbesondere VVOCs, VOCs und SVOCs unter den Bedingungen und mit den Möglichkeiten eines Werkslabors der Holzwerkstoffindustrie in kurzer Zeit, in weniger als 6 Std., erlauben.

Gelöst wird diese technische Problematik durch die unabhängigen Ansprüche. Vorteilhaften Ausgestaltungen der Erfindung können den Unteransprüchen entnommen werden.

Eine Analysevorrichtung gem. den Merkmalen des Anspruchs 1 ist zunächst äußerst kompakt und kann beispielsweise auf einem Labortisch problemlos Platz finden. Vorrichtungen zur kontrollierten Freisetzung von Gasen in wenigstens einer beheizbaren Prüfkammer sind wie auch gaschromatographische Säulen und Detektoren bekannt.

Gemäß der Erfindung ist in unmittelbarer Nachbarschaft der Vorrichtung ein Interface vorgesehen, in dem an einem Anreicherungsmedium eine Anreicherung der in der Prüfkammer der Vorrichtung freigesetzten und in einem Gasstrom austretenden Verbindungen erfolgt. Längere Schlauchleitungen oder dergleichen sind so vermieden. Eingebracht wird das Anreicherungsmedium in einen rohrförmigen Abschnitt des Interfaces, wozu ein gesondert ausgebildeter und dem Interface entnehmbarer Träger für das Anreicherungsmedium vorgesehen ist.

Als Anreicherungsmedien können Adsorbentien Verwendung finden, insbesondere Carboxen, oder auch Absorbentien, wie bspw. immobilisierte Flüssigkeiten, vorzugsweise Polydimethylsiloxan, PDMS, Polyethylenglycol, PEG, oder Polyamid, PA.

Des Weiteren ist das Interface beheizbar, so dass der Gasstrom wie auch das Anreicherungsmedium auf einem konstanten Temperaturniveau gehalten werden können. Auf Temperaturunterschieden basierende Messfehler werden durch diese Maßnahme verhindert.

Bei einer Ausführungsform einer Analysevorrichtung nach der Erfindung ist vorgesehen, dass eine Umsetzvorrichtung den Träger mit dem angereicherten Anreicherungsmedium in einen auf die gaschromatographische Säule aufgesetzten Injektor umsetzt.

In dem Injektor findet eine thermische Desorption statt, wonach in der gaschromatographischen Säule die Trennung der Substanzen erfolgen kann, die dann dem Detektor zugeführt werden.

Das Umsetzen des auf einem Träger aufgebrachten Anreicherungsmediums, beispielsweise einer Faser oder einer Platte, erfolgt vorzugsweise mit einem Kreuztisch der Umsetzvorrichtung, mit der der Träger horizontal und vertikal verfahren werden kann.

Weiter kann vorgesehen sein, dass der Träger in einer Schutzkanüle verfahrbar ist. Verunreinigungen bei dem Umsetzen des Trägers von dem Interface hin zu dem Injektor werden so vermieden.

Bei der Analysevorrichtung ist eine temperierbare Kalibriervorrichtung vorgesehen, die wenigstens ein Diffusions- und/oder Permeationsgefäß aufweist, so dass auch quantitative Aussagen ermöglicht sind. Die nötigen Kalibriergase oder Kalibriergasgemische können in herkömmlicher Weise durch Permeation oder Diffusion erzeugt werden.

Die Kalibrierung des Systems kann über die Verwendung externer und/oder interner Standards erfolgen.

Zur Verwendung externer Standards werden mittels Diffusion oder Permeation aus Reinsubstanzen Kalibriergase erzeugt und mittels der Kalibriervorrichtung auf das Anreicherungsmedium bzw. auf die SMPE-Faser oder einen anderen Träger des Anreicherungsmediums aufgegeben.

Zur Verwendung interner Standards werden mittels Diffusion oder Permeation aus Reinsubstanzen Kalibriergase erzeugt und mittels der Kalibriervorrichtung in die Prüfkammer der Analysevorrichtung eingebracht.

Die Auswahl der verwendeten Reinsubstanzen richtet sich nach den in realen Proben auftretenden und damit zu quantifizierenden Substanzen. Eine vereinfachte Version der Verwendung interner Standards sieht die Verwendung von 1,4-Cineol (CAS 470-67-7) zur Quantifizierung von Monoterpenen und -terpenoiden, und/oder von Aromadendren (CAS 489-39-4) zur Quantifizierung von Sesquiterpenen und -terpenoiden, und/oder von Ethylhexanoat (CAS 123-66-0) zur Quantifizierung von Aldehyden und Ketonen, und/oder Ethylfuran (CAS 3208-16-0) zur Quantifizierung sonstiger Substanzen vor. Die genannten Substanzen kommen nicht in Holz vor, ähneln jedoch strukturell den jeweiligen Zielsubstanzen und zeigen daher einen ähnlichen Response im Detektor.

Sowohl bei der Verwendung von externen als auch von internen Standards können Gemische von Kalibriergasen und/oder reine Kalibriergase erstellt und verwendet werden.

Das erfindungsgemäße Verfahren besteht im Wesentlichen aus vier Schritten. In einem ersten Schritt erfolgt die Ausgasung der Probe in der Prüfkammer der Vorrichtung und die Anreicherung eines Anreicherungsmediums auf einem einem Interface entnehmbaren Träger mit den in dem Gasstrom vorhandenen flüchtigen Verbindungen. Nach einem Umsetzen des Trägers mit dem Anreicherungsmedium folgt als zweiter Schritt dann eine thermische Desorption des angereicherten Anreicherungsmediums. In einem dritten Schritt erfolgt die Trennung der Substanzen in einer gaschromatographischen Säule und die Bestimmung der Substanzen in einem Detektor.

Hierzu ist das mit dem flüchtigen Verbindungen angereicherter Anreicherungsmedium von einer Umsetzvorrichtung für eine thermische Desorption in den auf eine gaschromatographische Säule aufgesetzten Injektor zu verbringen.

Nach dem Trennen der Substanzen in der gaschromatographischen Säule, die hierzu ein Temperaturprogramm durchläuft, erfolgt die Detektion mittels eines Field Asymmetrie Ion Mobility Spectrometer und folgt die Bestimmung der Substanzen durch einen Vergleich des gewonnenen Chromatogramms mit in einer Bibliothek hinterlegten Referenzwerten der Retentionszeit der Substanzen.

Das Wesen der Erfindung wird anhand der Zeichnung näher erläutert, in der lediglich schematisch Ausführungsbeispiele und Ergebnisse dargestellt sind. In der Zeichnung zeigt:
- Fig. 1:: die prinzipielle Anordnung einer Analysevorrichtung nach der Erfindung,
- Fig. 2:: in einem Schnitt ein Interface,
- Fig. 3:: ein unter Verwendung einer SPME-Faser vom Typ SPME fiber PDMS-DVB 65 um erhaltenes Chromatogramm,
- Fig. 4 bis 9:: die Peakhöhe der an der Faser angereicherten Substanzen in Abhängigkeit der Anreicherungszeit tₐₙᵣ und
- Fig. 10:: eine konkrete Ausgestaltung einer Umsetzvorrichtung.

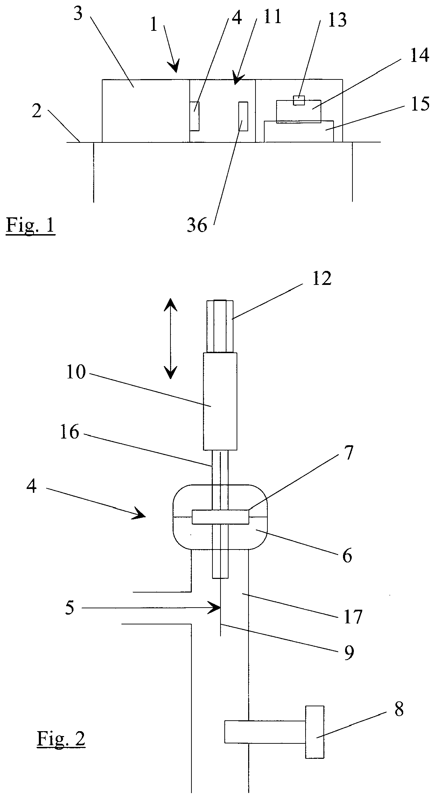

Die Analysevorrichtung 1 zeigt Fig. 1 beispielhaft auf einem Labortisch 2.

Dort ist weiter eine Vorrichtung 3 zur kontrollierten Freisetzung von insbesondere VVOCs, VOCs und SVOCs aus Holzwerkstoffen und anderen Holzprodukten dargestellt, z. B. eine nach DIN EN ISO 12460-3 arbeitende Gasanalyse-Anlage, hier mit einem nachstehend erläuterten automatisierten System zur Anreicherung, Trennung und Bestimmung der flüchtigen Verbindungen gekoppelt, z. B. einem SPME-Faden in einem Interface mit nachgeschalteter GC-Säule und einem Detektor, z. B. einem FAIMS, insbesondere einem High-Field AsymmetricWaveform Ion Mobility Spectrometer.

Zuerst wird ein Prüfkörper bspw. ein Holzwerkstoff oder ein Holzprodukt in einer Prüfkammer der Vorrichtung 3 zur kontrollierten Freisetzung von Gasen auf eine bestimmte Temperatur erwärmt (30°C bis 90°C). Die durch die Erwärmung des Prüfkörpers freigesetzten flüchtigen Verbindungen, insbesondere VVOCs, VOCs und SVOCs, werden mit Hilfe eines Gasstromes 5, vgl. Fig. 2, insbesondere aus gereinigter und konditionierter Luft, mit einem Volumenstrom von 20 bis 80 l/h, vorzugsweise von 60 l/h, aus der Prüfkammer der Vorrichtung 3 herausgeführt.

Am Ausgang der Prüfkammer befindet sich ein beheiztes Interface 4, vgl. Fig. 2, in dem in einem nächsten Verfahrensschritt eine Anreicherung (Sammlung) der freigesetzten Verbindungen stattfindet. Das Interface 4 beinhaltet eine Verschraubung 6 mit einer Dichtung 7, vorzugsweise einem Septum, und eine manuelle oder automatisierte Stellschraube, gegebenenfalls ein Ventil 8 zur Aufrechterhaltung eines konstanten Druckes in der Prüfkammer der Vorrichtung.

Die Anreicherung eines Anreicherungsmediums auf einem Träger 9, bei diesem Ausführungsbeispiel eine SPME-Faser, vorzugsweise PDMS/DVB, gegebenenfalls auch eine mit dem Anreicherungsmedium belegten Platte, erfolgt in einer senkrechten Anordnung zu dem Gasstrom 5 in einem rohrförmigen Abschnitt 17 des Interfaces 4.

Die Anreicherung kann bei 0 min beginnen, mit Einbringen des Prüfkörpers in die Prüfkammer bzw. dem Beginn der Prüfung, oder später, vorzugsweise bei 105 min. Die Dauer des Anreicherungsschrittes ist zwischen 0,1 und 60 Minuten frei wählbar und liegt vorzugsweise bei 30 Minuten.

Mit Abschluss der Anreicherung wird die von einem Faserhalter 10 gehaltene SPME-Faser 9 von bspw. einem Kolben 12 in eine Schutzkanüle 16 eingezogen und wird der SPME-Faserhalter 10 mit der SPM-Faser 9 von einer Umsetzvorrichtung 11 automatisiert in einen heißen Injektor 13 gem. Fig. 1 überführt, dessen Temperatur zwischen 200 und 280°C liegt, vorzugsweise bei 250°C. In diesem Injektor 13 findet eine thermische Desorption, ein Freisetzen der zuvor angereicherten Verbindungen, statt.

Nach der thermischen Desorption im Injektor 13 erfolgt die Trennung der Substanzen in einer gaschromatographischen Säule 14 (GC-Säule). Es können alle kommerziell erhältlichen Säulentypen, wie z.B. FusedSilica, Metallkapillar oder Multikapillarsäulen (MCC), Verwendung finden.

Die Säulenlänge kann zwischen 1 und 60 m variieren. Der innere Durchmesser kann zwischen 0,1 und 1,0 mm betragen. Die Beschichtung der inneren Phase (Trennphase) kann zwischen 0,1 und 7 um betragen. Es können sowohl unpolare, schwach bis mittelpolare als auch stark polare Säulen zum Einsatz kommen.

Typisch ist bspw. eine Säulenlänge von 30 m, ein innerer Durchmesser von 0,25 mm und eine leichtpolare Trennphase mit einer Beschichtungsstärke von 0,25 µm.

Der Trägergasfluss (Säulenfluss) kann in Abhängigkeit von der verwendeten GC-Säule 14 zwischen 0,5 und 20 ml/min betragen. Als Trägergase kommen neben insbesondere aufbereiteter Luft Inertgase wie Helium oder Stickstoff zum Einsatz.

Zur Optimierung der Trennung der Substanzen hinsichtlich der zeitlichen Auflösung kann die GC-Säule 14 einem Temperaturprogramm unterworfen werden oder auch isotherm gehalten werden. Die Beheizung der GC-Säule 14 kann dabei direkt mittels Heizdrahtes (Widerstandsheizung) erfolgen oder indirekt mittels GC-Ofen mit Luft als Wärmeübertragungsmedium.

Ein Beispiel für ein Temperaturprogramm: Start bei 40°C für 2 min, dann Aufheizen mit 5°C/min auf 200°C, anschließend 20 min isotherm.

Alternativ: Start zwischen 30 und 80°C; bis zu 100°C pro Minute aufheizen, isothermer Haltepunkt bei 120°C (für 1-20 min); anschließend weitere Rampe bis zu 250°C.

Mit einer Kryofokussierung sind Starttemperaturen von weit unter 0°C möglich, bspw. durch Einsatz von flüssigem Stickstoff oder Peltierelementen. Eine solche Kryofokussierung wird standardmäßig in der Referenzmethode (Thermodesorption-GC-MS) verwendet.

Die anschließende Detektion erfolgt vorzugsweise mittels eines Field Asymmetric Ion Mobility Spectrometer 15, FAIMS, insbesondere eines High-Field Asymetric Ion Mobility Spectrometer, und kann sowohl mit einer radioaktiven Quelle (Ni-63) als auch mit einer nicht radioaktiven Quelle als Ionisator wie z. B. einer UV-Lampe mit einer Ionisierungsenergie zwischen 8 und 12 eV versehen werden.

### Parameter:

Messen mit einer konstanten oder variablen Hochspannung. Die Hochspannung kann dabei zwischen 0 - 100% (entspricht 0 - ca. 80 kV) betragen im Gegensatz zu bisherigen 30 bis 40%

Der Trägergasstrom für das FAIMS kann zwischen 0,1 und 5 l/min betragen statt bisher 1,6 l/min.

Die Kompensationsspannung kann zwischen -6 Vdc und + 6Vdc betragen statt bisher zwischen -3 Vdc bis +3 Vdc.

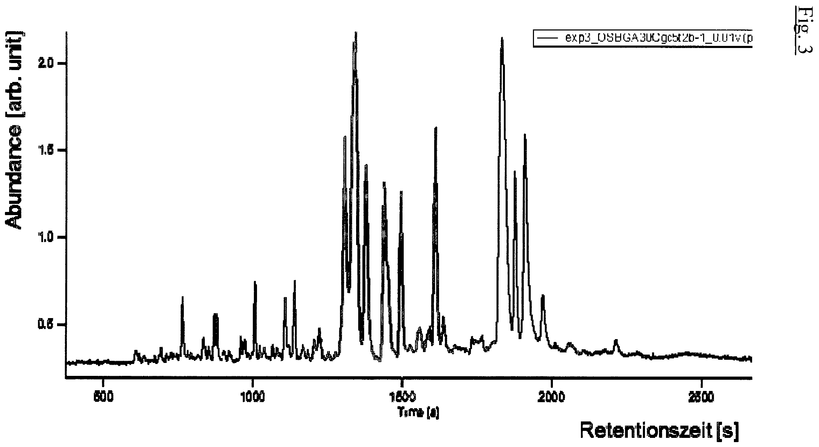

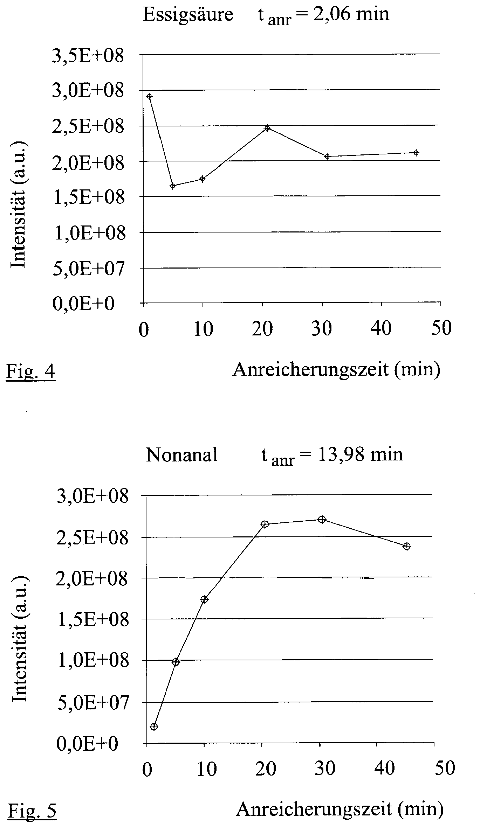

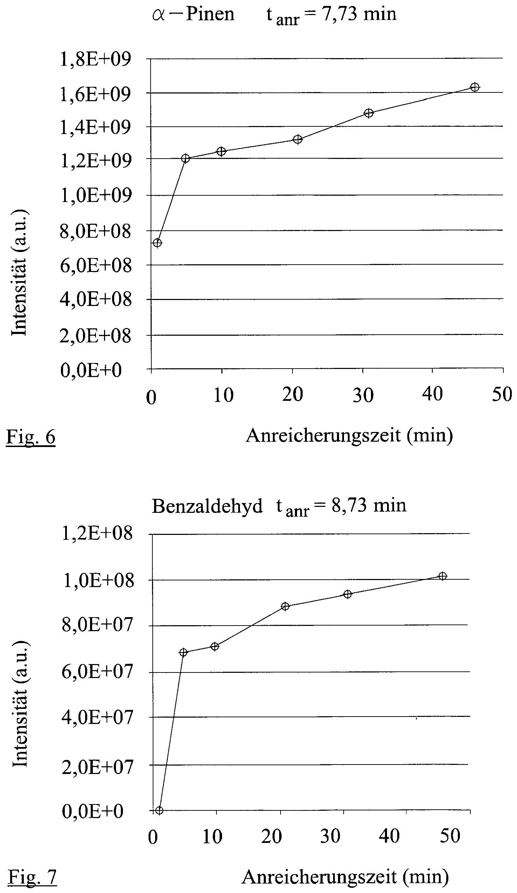

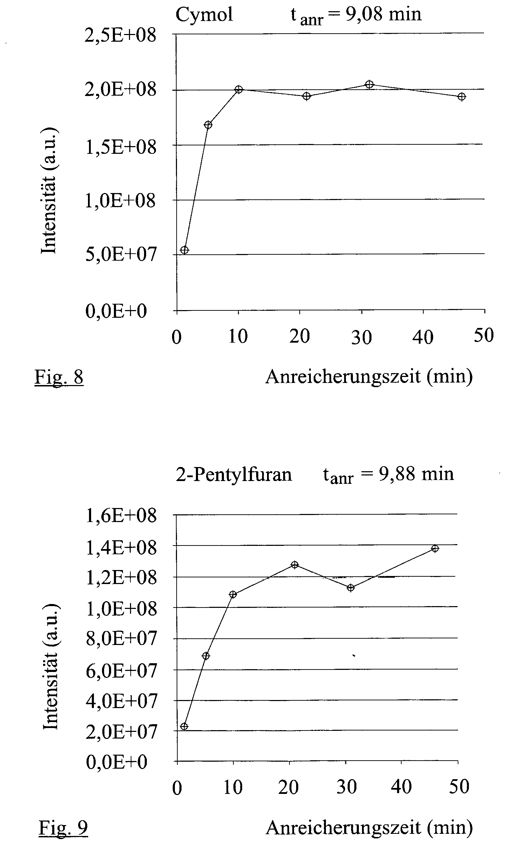

Als Anreicherungsmedium haben sich SPME-Fasern 9 bewährt, insbesondere vom Typ SPME fiber PDMS-DVB 65 um. Ein Beispiel-Chromatogramm, das unter Verwendung dieses SPME-Fasertyps aufgenommen wurde, ist in Fig. 3 wiedergegeben. Die Zuordnung der relativen Maxima (Peaks) zu den detektierten Stoffen erfolgt durch einen Vergleich mit in einer Bibliothek hinterlegten Daten. Die Fig. 4 bis 9 zeigen beispielhaft die Peakhöhe der an der Faser angereicherten Substanzen in Abhängigkeit der Anreicherungszeit tₐₙᵣ.

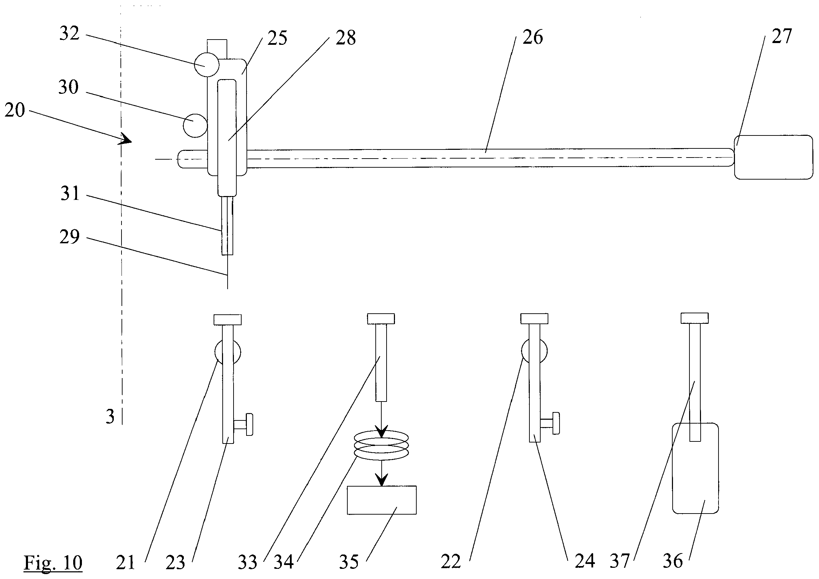

Anhand der Figur 10 wird ein konkretes Ausführungsbeispiel einer Umsetzvorrichtung 20 vor oder an einer angedeuteten Vorrichtung 3 erläutert.

Die Vorrichtung 3 weist zwei Prüfkammern auf mit jeweils einer Austrittsöffnung 21,22. Vor jeder Austrittsöffnung 21,22 ist jeweils ein Interface 23,24 gem. Figur 2 vorgesehen. Oberhalb der Interfaces 23,24 ist ein Schlitten 25 an einer Schiene 26 horizontal verfahrbar angeordnet, beispielsweise angetrieben durch einen Schrittmotor 27, der eine sehr genaue und einfache Positionierung des Schlittens 25 über den Interfaces 23,24 erlaubt.

Auf dem Schlitten 25 ist ein Faserhalter 28 für eine SPME-Faser 29 nach Art eines Kreuztisches senkrecht verfahrbar angeordnet, beispielsweise mittels eines Elektromotors 30. Für eine vertikale Bewegung der in einer Schutzkanüle 31 gefangenen SPME-Faser 29 ist ein weiterer Stellmotor 32 vorgesehen. Ebenfalls unter der Umsetzvorrichtung 20 angeordnet ist ein Injektor 33, in den nach erfolgter Anreicherung das Anreicherungsmedium auf dem SPME-Faden 29 dieser von der Umsetzvorrichtung 20 umgesetzt wird.

Nach der thermischen Desorption in dem Injektor 32 erfolgt die Trennung der Verbindungen in einer gaschromatographischen Säule 34 mit anschließender Detektion der Substanzen in einem Detektor, einem FAIMS 35.

Um neben einer qualitativen Aussage auch eine quantitative vornehmen zu können, zeigt die Figur 10 ferner eine Kalibriervorrichtung 36 mit einer den Interfaces 23,24 ähnlichen Aufnahme 37, in die die Umsetzvorrichtung 20 ebenfalls eine SPME-Faser einführen kann.

### Bezugszeichenliste:

1. Analysevorrichtung
2. Labortisch
3. Vorrichtung
4. Interface
5. Gasstrom
6. Verschraubung
7. Dichtung
8. Stellschraube, Ventil
9. Träger, SPME-Faser
10. Faserhalter
11.Umsetzvorrichtung
12. Kolben
13. Injektor
14.gaschromatographische Säule
15.Detektor
16.Schutzkanüle
17. Abschnitt
18.
19.
20. Umsetzvorrichtung
21.Austrittsöffnung
22.Austrittsöffnung
23. Interface
24. Interface
25.Schlitten
26.Schiene
27.Schrittmotor
28.Faserhalter
29.Träger,
   SPME-Faser
30.Elektromotor
31.Schutzkanüle
32.Stellmotor
33.Injektor
34.GC-Säule
35.FAIMS
36.Kalibriervorrichtung
37.Aufnahme

## Patentansprüche

1. Analysevorrichtung (1) für die Durchführung einer Gasanalyse, aufweisend eine Vorrichtung (3) zur kontrollierten Freisetzung von Gasen aus einer Probe, wenigstens ein beheizbares Interface (4) und eine gaschromatographischen Säule (14) mit einem nachgeschalteten Detektor (15), wobei an die Vorrichtung (3) das wenigstens eine beheizbare Interface (4) angeschlossen ist, **dadurch gekennzeichnet, dass** in dem Interface (4) eine Anreicherung der in einer Prüfkammer der Vorrichtung (3) freigesetzten und in einem Gasstrom (5) austretenden Verbindungen an einem Anreicherungsmedium auf einem Träger (9) in einem rohrförmigen Abschnitt (17) erfolgt, wobei der Träger (9) senkrecht zu dem Gasstrom (5) angeordnet ist, und dass der dem Interface (4) entnehmbaren Träger (9) durch eine SPME-Faser (9) oder eine mit dem Anreicherungsmedium belegte Platte ausgebildet wird.

2. Analysevorrichtung nach Anspruch 1, **dadurch gekennzeichnet, dass** durch das Interface (4) ein konstanter Druck in der Vorrichtung (3) aufrecht erhalten wird.

3. Analysevorrichtung nach einem oder mehreren der vorangehenden Ansprüche, **dadurch gekennzeichnet, dass** das Interface (4) eine Dichtung (7) oder ein Septum aufweist.

4. Analysevorrichtung nach einem oder mehreren der vorangehenden Ansprüche, **dadurch gekennzeichnet, dass** eine Umsetzvorrichtung (20) den Träger (29) mit dem angereicherten Anreicherungsmedium in einen auf die gaschromatographische Säule (34) aufgesetzten Injektor (33) umsetzt.

5. Analysevorrichtung nach einem oder mehreren der vorangehenden Ansprüche, **dadurch gekennzeichnet, dass** der Träger (29) an einem Kreuztisch der Umsetzvorrichtung (20) horizontal und vertikal verfahrbar ist.

6. Analysevorrichtung nach einem oder mehreren der vorangehenden Ansprüche, **dadurch gekennzeichnet, dass** der Träger (9) in einer Schutzkanüle (31) verfahrbar ist.

7. Analysevorrichtung nach einem oder mehren der vorangehenden Ansprüche, **dadurch gekennzeichnet, dass** eine temperierbare Kalibriervorrichtung (36) vorgesehen ist, die wenigstens ein Diffusions- und/oder Permeationsgefäß aufweist.

8. Analysevorrichtung nach Anspruch 9, **dadurch gekennzeichnet, dass** mit der Kalibriervorrichtung (36) eine externe und/oder interne Kalibrierung erfolgen kann.

9. Analysevorrichtung nach einem oder mehreren der vorangehenden Ansprüche, **dadurch gekennzeichnet, dass** in dem Interface oder dem Injektor eine thermische Desorption erfolgt.

10. Verfahren der Gasanalyse, bei der eine Probe einer Wärmebehandlung für ein Ausgasen unterzogen wird, **dadurch gekennzeichnet, dass** in einem Interface (4) ein Anreicherungsmedium (9) auf einem dem Interface (4) entnehmbaren Träger (9) mit flüchtigen Verbindungen eines Gasstroms (5) in einer senkrechten Anordnung zu dem Gasstrom (5) angereichert wird, dass der durch eine SPME-Faser oder eine mit dem Anreicherungsmedium belegte Platte ausgebildete Träger (9) mit dem Anreicherungsmedium umgesetzt einer thermische Desorption unterzogen wird, dass in einer gaschromatographischen Säule (14) eine Trennung der Substanzen erfolgt und dass anschließend die Bestimmung der Substanzen in einem Detektor (15) erfolgt.

11. Verfahren der Gasanalyse nach Anspruch 10, **dadurch gekennzeichnet, dass** der Träger (9) mit den flüchtigen Verbindungen angereichertes Anreicherungsmedium (9) von einer Umsetzvorrichtung (11) für eine thermische Desorption in einen auf eine gaschromatographischen Säule (14) aufgesetzten Injektor (13) verbracht wird.

12. Verfahren nach Anspruch 10 oder 11, **dadurch gekennzeichnet, dass** nach der Trennung der Substanzen in der gaschromatographischen Säule (50,62) eine Detektion mit einem Field Asymmetrie Ion Mobility Spectrometer (51,63) vorgesehen ist und dass die Bestimmung der Substanzen durch einen Vergleich des gewonnen Chromatogramms mit in einer Bibliothek hinterlegten Referenzwerten der Retentionszeit (tᵣₑₜ) der Substanzen erfolgt.

13. Verfahren nach einem oder mehreren der Ansprüche 10 bis 12, **dadurch gekennzeichnet, dass** für eine Kalibrierung mit einem Kalibriergasgemisch als Reinsubstanzen 1,4-Cineol und/oder Aromadendren und/oder Ethylhexanoat und/oder 2-Ethylfuran Verwendung finden.

## Claims

1. An analysis device (1) for performing a gas analysis, comprising a device (3) for the controlled release of gases from a sample, at least one heatable interface (4) and a gas chromatographic column (14) with a downstream detector (15), wherein the at least one heatable interface (4) is connected to the device (3), **characterised in that** an enrichment of the compounds released in a test chamber of the device (3) and exiting in a gas flow (5) with an enrichment medium on a carrier (9) takes place in a tubular section (17) in the interface (4), wherein the carrier (9) is arranged perpendicular to the gas flow (5), and that the carrier (9) which can be removed from the interface (4) is formed by an SPME fibre (9) or a plate coated with the enrichment medium.

2. The analysis device according to claim 1, **characterised in that** a constant pressure is maintained in the device (3) by the interface (4).

3. The analysis device according to one or more of the preceding claims, **characterised in that** the interface (4) comprises a seal (7) or a septum.

4. The analysis device according to one or more of the preceding claims, **characterised in that** a conversion device (20) converts the carrier (29) with the enriched enrichment medium into an injector (33) placed on the gas chromatographic column (34).

5. The analysis device according to one or more of the preceding claims, **characterised in that** the carrier (29) can be moved horizontally and vertically on a cross table of the conversion device (20).

6. The analysis device according to one or more of the preceding claims, **characterised in that** the carrier (9) can be moved in a protective tube (31).

7. The analysis device according to one or more of the preceding claims, **characterised in that** a temperature-regulatable calibration device (36) is provided, which comprises at least one diffusion and/or permeation vessel.

8. The analysis device according to claim 9, **characterised in that** an external and/or internal calibration can take place with the calibration device (36).

9. The analysis device according to one or more of the preceding claims, **characterised in that** a thermal desorption takes place in the interface or the injector. I

10. A method for gas analysis, in which a sample is subjected to a heat treatment for outgassing, **characterised in that** an enrichment medium (9) on a carrier (9) which can be removed from the interface is enriched (4) in the interface (4) with volatile compounds of a gas flow (5) in a perpendicular arrangement to the gas flow (5), that the carrier (9) constituted by an SPME fibre or a plate coated with the enrichment medium is subjected, converted with the enrichment medium, to a thermal desorption, that a separation of the substances takes place in the gas chromatographic column (14) and that the determination of the substances then takes place in a detector (15).

11. The method for gas analysis according to claim 10, **characterised in that** the carrier (9) with the enrichment medium (9) enriched with the volatile compounds is brought from a conversion device (11) for a thermal desorption into an injector (13) placed on a gas chromatographic column (14).

12. The method according to claim 10 or 11, **characterised in that**, after the separation of the substances in the gas chromatographic column (50, 62), a detection with a field asymmetric ion mobility spectrometer (51, 63) is provided and that the determination of the substances takes place by a comparison of the obtained chromatograph with reference values of the retention time (tᵣₑₜ) of the substances stored in a library.

13. The method according to one or more of claims 10 to 12, **characterised in that** 1,4-cineole and/or aromadendrene and/or ethyl hexanoate and/or 2-ethylfuran are used as pure substances for a calibration with a calibration gas mixture.

## Revendications

1. Dispositif d'analyse (1), destiné à réaliser une analyse des gaz, comportant un dispositif (3) pour la libération contrôlée de gaz à partir d'un échantillon, au moins une interface (4) pouvant être chauffée et une colonne de chromatographie gazeuse (14) pourvue d'un détecteur (15) monté en aval, sur le dispositif (3) étant raccordée l'au moins une interface (4) pouvant être chauffée, **caractérisé en ce que** dans l'interface (4) s'effectue un enrichissement d'un milieu d'enrichissement en composés libérés dans une chambre d'essai du dispositif (3) et s'échappant dans un flux gazeux (5) sur un support (9) dans une partie (17) de forme tubulaire, le support (9) étant placé à la perpendiculaire du flux gazeux (5) et **en ce que** le support (9) pouvant être retiré de l'interface (4) est constitué d'une fibre SPME (9) ou d'une plaque recouverte du milieu d'enrichissement.

2. Dispositif d'analyse selon la revendication 1, **caractérisé en ce que** par l'interface (4), une pression constante est maintenue dans le dispositif (3) .

3. Dispositif d'analyse selon l'une quelconque des revendications précédentes, **caractérisé en ce que** l'interface (4) comporte un joint (7) ou un septum.

4. Dispositif d'analyse selon l'une quelconque des revendications précédentes, **caractérisé en ce qu'**un dispositif de transfert (20) transfère le support (29) avec le milieu d'enrichissement enrichi dans un injecteur (33) posé sur la colonne de chromatographie (34) .

5. Dispositif d'analyse selon l'une quelconque des revendications précédentes, **caractérisé en ce que** le support (29) est déplaçable à l'horizontale et à la verticale sur une platine à chariot du dispositif de transfert (20).

6. Dispositif d'analyse selon l'une quelconque des revendications précédentes, **caractérisé en ce que** le support (9) est déplaçable dans une canule protectrice (31) .

7. Dispositif d'analyse selon l'une quelconque des revendications précédentes, **caractérisé en ce qu'**il est prévu un dispositif d'étalonnage (36) tempérable qui comporte au moins un vase de diffusion et / ou de perméation.

8. Dispositif d'analyse selon la revendication 9, **caractérisé en ce qu'**avec le dispositif d'étalonnage (36) peut s'effectuer un étalonnage externe et / ou interne.

9. Dispositif d'analyse selon l'une quelconque des revendications précédentes, **caractérisé en ce que** dans l'interface ou dans l'injecteur s'effectue une désorption thermique.

10. Procédé d'analyse des gaz, lors duquel l'on soumet un échantillon à un traitement thermique pour un dégazage, **caractérisé en ce que** dans une interface (4), l'on enrichit un milieu d'enrichissement (9) sur un support (9) pouvant être retiré de l'interface (4) en composés volatils d'un flux gazeux (5), en placement perpendiculaire par rapport au flux gazeux (5), **en ce que** l'on soumet le support (9) constitué d'une fibre SPME ou d'une plaque recouverte du milieu d'enrichissement transféré avec le milieu d'enrichissement à une désorption thermique, **en ce que** dans une colonne de chromatographie gazeuse (14) s'effectue une séparation des substances et **en ce que** par la suite, la détermination des substances s'effectue dans un détecteur (15).

11. Procédé d'analyse des gaz selon la revendication 10, **caractérisé en ce que** pour une désorption thermique, par un dispositif de transfert (11), le support (9) est amené avec le milieu d'enrichissement (9) enrichi avec les composés volatils dans un injecteur (13) posé sur une colonne de chromatographie (14).

12. Procédé selon la revendication 10 ou 11, **caractérisé en ce qu'**après la séparation des substances dans la colonne de chromatographie gazeuse (50, 62), il est prévu une détection avec un spectromètre de mobilité ionique à champ asymétrique (51, 63) et **en ce que** la détermination des substances s'effectue par comparaison du chromatogramme obtenu avec des valeurs de référence du temps de rétention (t = t) des substances, sauvegardées dans une bibliothèque.

13. Procédé selon l'une quelconque ou plusieurs des revendications 10 à 12 précédentes, **caractérisé en ce que** pour un étalonnage avec un mélange de gaz d'étalonnage, les substances pures utilisées sont le 1,4-cinéol et/ou l'aromadendrène et/ou l'éthylhexanoate et/ou le 2-ethylfuran.
